(19) Europäisches Patentamt
European Patent Office
Office européen des brevets

(11) Veröffentlichungsnummer : **0 043 971**
**B1**

(12) # EUROPÄISCHE PATENTSCHRIFT

(45) Veröffentlichungstag der Patentschrift : **19.12.84**

(51) Int. Cl.$^3$ : **C 07 D295/08**, A 61 K 31/495

(21) Anmeldenummer : **81105028.5**

(22) Anmeldetag : **29.06.81**

(54) **Indanon-oxyalkyl-piperazinderivate, ihre Herstellung und diese enthaltende pharmazeutische Zubereitungen.**

(30) Priorität : **11.07.80 DE 3026331**

(43) Veröffentlichungstag der Anmeldung : **20.01.82 Patentblatt 82/03**

(45) Bekanntmachung des Hinweises auf die Patenterteilung : **19.12.84 Patentblatt 84/51**

(84) Benannte Vertragsstaaten : **AT BE CH DE FR GB IT LI LU NL SE**

(56) Entgegenhaltungen :
**DE-A- 2 116 213**
**DE-A- 2 335 432**
**DE-A- 2 633 214**
**DE-A- 2 824 677**
**Die Akte enthält technische Angaben die nach dem Eingang der Anmeldung eingereicht wurden und die nicht in dieser Patentschrift enthalten sind.**

(73) Patentinhaber : **BASF Aktiengesellschaft**
**Carl-Bosch-Strasse 38**
**D-6700 Ludwigshafen (DE)**

(72) Erfinder : **Franke, Albrecht, Dr.**
**Mandelring 11**
**D-6706 Wachenheim (DE)**
Erfinder : **Lenke, Dieter, Dr.**
**Kekuleplatz 1**
**D-6700 Ludwigshafen (DE)**
Erfinder : **Gries, Josef, Dr.**
**Roemerweg 43**
**D-6706 Wachenheim (DE)**
Erfinder : **Weifenbach, Harald, Dr.**
**Londoner Ring 71**
**D-6700 Ludwigshafen (DE)**
Erfinder : **Hofmann, Hans Peter, Dr.**
**Untere Hart 12**
**D-6703 Limburgerhof (DE)**
Erfinder : **Kreiskott, Horst, Dr.**
**Am Boehlig**
**D-6706 Wachenheim (DE)**

**EP 0 043 971 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die vorliegende Erfindung betrifft neue Indanon-oxyalkylpiperazinderivate und ihre Säureadditionssalze, deren Herstellung und diese Verbindungen enthaltende pharmazeutischen Zubereitungen, die als Sedativa, Tranquilizer, Neuroleptica oder bei der Behandlung von Hypertonie verwendet werden können.

In der DE-OS 23 35 432 wird beispielsweise beschrieben, daß 3,4-Dihydro-2H-naphthalin-1-on-5-oxypropyl-piperazin-derivate blutdrucksenkende, antiödematöse, antiallergische und sedierende Eigenschaften aufweisen. Ihre Wirkung befriedigt jedoch nicht immer.

Es wurde nun gefunden, daß Verbindungen der allgemeinen Formel (I)

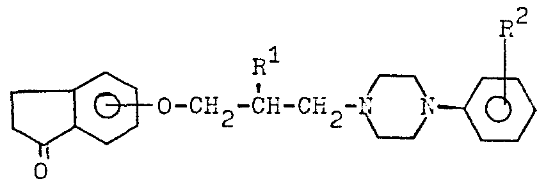

(I)

in der

$R^1$ ein Wasserstoffatom oder eine Hydroxygruppe und

$R^2$ ein Chlor- oder Fluoratom, eine Methoxy- oder Methylgruppe bedeuten

und die Oxyalkylpiperazinseitenkette in der 4-, 5- oder 7-Stellung des Indanon-1-ringsystems sich befindet, und ihre physiologisch verträglichen Säureadditionssalze sich durch wertvolle pharmazeutische Eigenschaften auszeichnen.

Als erfindungsgemäße Verbindungen können neben den in den Beispielen angegebenen Verbindungen die folgenden beispielsweise genannt werden :

5-(3-[4-(4-Fluorphenyl)-1-piperazinyl]-2-hydroxy-propoxy)-indanon-1
5-(3-[4-(4-Chlorphenyl)-1-piperazinyl]-2-hydroxy-propoxy)-indanon-1
5-(3-[4-(3-Chlorphenyl)-1-piperazinyl]-2-hydroxy-propoxy)-indanon-1
5-(3-[4-(4-Fluorphenyl)-1-piperazinyl]-propoxy)-indanon-1
5-(3-(4-(4-Chlorphenyl)-1-piperazinyl]-propoxy)-indanon-1
7-(3-[4-(3-Chlorphenyl)-1-piperazinyl]-propoxy)-indanon-1

Die erfindungsgemäßen Verbindungen werden hergestellt, indem man ein Indanon-Derivat der Formel (II)

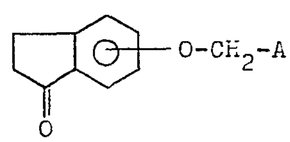

(II)

in der A den Rest

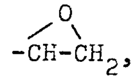, 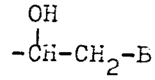 oder $-CH_2-E$

bedeutet und wobei B für eine nukleofuge Abgangsgruppe steht, mit einem Piperazinderivat der allgemeinen Formel (III)

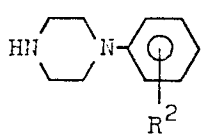

(III)

in der $R^2$ die für die Formel (I) angegebenen Bedeutungen hat, zweckmäßigerweise in einem Lösungsmittel und ggf. in Gegenwart eines säurebindenden Mittels in an sich bekannter Weise umsetzt und die erhaltene Verbindung ggf. in das Säureadditionssalz einer physiologisch verträglichen Säure überführt.

Die Abgangsgruppe B stellt bevorzugt ein Halogenatom, insbesondere Chlor, Brom oder Jod, dar. Weiterhin kommen beispielsweise als nukleofuge Abgangsgruppen aromatische oder aliphatische Sulfonsäurereste in Betracht, wie der p-Toluolsulfonsäure-, der p-Brombenzolsulfonsäure- oder der Methansulfonsäurerest.

Die Umsetzungen werden bei Temperaturen von 10 bis 120 °C, d. h. bei Raumtemperaturen oder bei höheren Temperaturen, zweckmäßig bei Temperaturen von 50 bis 120 °C, durchgeführt. Die Umsetzungen können unter Atmosphärendruck oder in einem geschlossenen Gefäß unter erhöhtem Druck ggf. unter Erwärmung auf den angegebenen Temperaturbereich durchgeführt werden.

Die Ausgangsverbindungen können direkt, d. h. ohne Zugabe eines Verdünnungs- oder Lösungsmittels, umgesetzt werden. Zweckmäßigerweise werden die Umsetzungen jedoch in Gegenwart eines inerten Verdünnungs- oder Lösungsmittels, beispielsweise eines niederen Alkohols mit 1 bis 4 C-Atomen, wie Methanol, Äthanol oder Propanol, vorzugsweise Isopropanol oder Äthanol, eines niederen gesättigten Dialkyläthers, Dialkylglykoläthers oder cyclischen Äthers, wie Diäthyläther, 1,2-Dimethoxyäthan, Tetrahydrofuran oder Dioxan, eines aromatischen Kohlenwasserstoffes, wie Benzol oder eines Alkylbenzols, wie Toluol oder Xylol, oder eines gesättigten aliphatischen Kohlenwasserstoffes, wie Hexan, Heptan oder Octan, eines niederen aliphatischen Ketons, wie Aceton, Methyläthylketon oder Methyl-isobutylketon, eines Dialkylformamids, wie Dimethyl- oder Diäthylformamid, oder von Dimethylsulfoxid oder in Gegenwart von Wasser oder in Mischungen der genannten Lösungsmittel, durchgeführt.

Bevorzugte Lösungsmittel bei der Umsetzung eines Epoxids der Formel (II), beispielsweise von 1-(Indan-1-on-4-oxy)-2,3-epoxypropan, 1-(Indan-1-on-5-oxy)-2,3-epoxypropan, 1-(Indan-1-on-6-oxy)-2,3-epoxypropan oder 1-(Indan-1-on-7-oxy)-2,3-epoxypropan mit einem Piperazin der allgemeinen Formel (III) sind niedere Alkohole, insbesondere n-Propanol, wobei die Umsetzung bevorzugt bei Temperaturen von 50 bis 120 °C und bei Normaldruck durchgeführt wird.

Bei der nukleophilen Substitution eines Restes B in einer Verbindung der Formel (II) mit einem Rest $—CH_2—B$, beispielsweise von 1-(Indan-1-on-4-oxy)-3-chlorpropan oder 1-(Indan-1-on-7-oxy)-3-chlorpropan mit einem Piperazin-derivat der allgemeinen Formel (III) sind als Lösungsmittel ein niederes aliphatisches Keton, wie Aceton, Diäthylketon, Methylisopropylketon oder Methylisobutylketon, ein cyclischer gesättigter Äther, insbesondere Tetrahydrofuran oder Dioxan, oder ein Dialkylformamid, wie Dimethylformamid, und Temperaturen von 50 bis 120 °C bevorzugt. Gegebenenfalls wird dabei die Reaktion in Gegenwart einer katalytischen Menge Natrium- oder Kaliumjodid durchgeführt.

Es sei erwähnt, daß als Ausgangsverbindung der Formel (II) gegebenenfalls auch eine Mischung des Epoxids mit einem Halogenhydrin in Betracht kommt, da bei der technischen Herstellung der Ausgangsverbindungen der Formel II derartige Gemische unter Umständen entstehen können.

Bei einer zweckmäßigen Ausführungsform zur nukleophilen Substitution des Restes B durch das verwendete Piperazinderivat wird die Umsetzung in Gegenwart einer Base als säurebindendes Mittel durchgeführt. Bevorzugte Basen sind Alkalimetallhydroxyde, -carbonate, -hydrogencarbonate, -alkoholate oder ein tertiäres organisches Amin, wie Pyridin oder ein Trialkylamin, wie Trimethylamin oder Triäthylamin. Von den Alkaliverbindungen kommen insbesondere die des Natriums oder Kaliums in Betracht. Dabei wird die Base in stöchiometrischer Menge oder in geringem Überschuß verwendet. Gegebenenfalls ist es zweckmäßig, das zur Umsetzung verwendete Piperazinderivat (III) in überschüssiger Menge gleichzeitig als säurebindendes Mittel zu verwenden.

Die vollständige Umsetzung hängt von der Reaktionstemperatur ab und ist im allgemeinen innerhalb von 2 bis 15 Stunden beendet. Das Reaktionsprodukt kann in an sich üblicher Weise gewonnen werden, z. B. durch Filtration oder Abdestillieren des Verdünnungs- oder Lösungsmittels aus dem Reaktionsgemisch. Eine Reinigung der erhaltenen Verbindung erfolgt in üblicher Weise, beispielsweise durch Umkristallisieren aus einem Lösungsmittel, überführen in eine Säureadditionsverbindung oder durch Säulenchromatographie.

Die Ausgangsverbindungen der Formel (II) können durch Alkylierung des der Verbindung zugrundeliegenden Hydroxy-indanons, wie 4-Hydroxy-indan-1-on, 5-Hydroxy-indan-1-on, und 7-Hydroxy-indan-1-on, mit einem Epihalogenhydrin, einem α,ω-Dihalogen-2-propanol oder α,ω-Dihalogenpropan erhalten werden. Als Epihalogenhydrine kommen Epichlorhydrin, Epibromhydrin und Epijodhydrin, als α,ω-Dihalogen-2-propanole kommen insbesondere 1,3-Dichlor-2-propanol und 1,3-Dibrom-2-propanol und als α,ω-Dihalogenpropane insbesondere 1,3-Chlorbrompropan ; 1,3-Dichlorpropan oder 1,3-Dibrompropan in Betracht.

Die Herstellung der zu verwendenden Hydroxy-indan-1-one ist literaturbekannt (Organic Reactions, Band II, S. 114-117 ; J.D. Loudon, R.K. Razdan, J. Chem. Soc. *1954*, S. 4299-4303).

Die Alkylierung der Hydroxy-indan-1-one zur Herstellung der Ausgangsverbindungen der Formel (II) werden zweckmäßigerweise bei Temperaturen von 50 bis 120 °C und unter Normaldruck oder in einem geschlossenen Gefäß unter erhöhtem Druck durchgeführt. Die Umsetzungen werden zweckmäßigerweise in einem inerten Verdünnungs- oder Lösungsmittel, z. B. einen niederen aliphatischen Keton, wie Aceton, Methyläthylketon oder Methylisobutylketon, einem niederen Alkohol mit 1 bis 4 C-Atomen, wie Methanol, Äthanol, Propanol oder Butanol, einem niederen Alkylacetat, wie Methyl-, Äthyl- oder Propylacetat, einem Dialkylformamid, wie Dimethylformamid oder Diäthylformamid, oder Dimethylsulfoxid oder mit überschüssigem Alkylierungsmittel als Verdünnungs- oder Lösungsmittel durchgeführt.

Bevorzugt werden die Umsetzungen in Gegenwart einer Base als säurebindendem Mittel vorgenommen. Geeignete Basen sind Alkalimetallcarbonate, -hydrogencarbonate, hydroxide oder -alkoholate, insbesondere des Natriums und Kaliums, basische Oxide, wie Aluminiumoxid oder Calciumoxid, organische tertiäre Basen, wie Pyridin oder niedere Trialkylamine, wie Trimethyl- oder Triäthylamin.

Dabei können die Basen im Verhältnis zum eingesetzten Alkylierungsmittel in katalytischer Menge oder in stöchiometrischer Menge bzw. in geringem Überschuß verwendet werden.

Bevorzugt werden die Hydroxy-1-indan-one mit Epichlorhydrin oder Epibromhydrin, 1,3-Dibrompropanol-2 oder 1,3-Dibrompropan in einem niederen aliphatischen Keton, insbesondere Aceton oder Methyl-isobutylketon, in Gegenwart von mindestens einem Moläquivalent Base, insbesondere Kaliumcarbonat, bezogen auf das Alkylierungsmittel, bei Temperaturen von 50 bis 80 °C umgesetzt.

Weiterhin sei erwähnt, daß die Ausgangsverbindungen der Formel (II) mit einer Epoxygruppe oder mit Halogenhydrinstruktur durch einfache Säure-Base-Reaktion ineinander umgewandelt werden können. So läßt sich ein 1-(Indan-1-on-oxy)-2,3-epoxypropan mit der entsprechenden Halogenwasserstoffsäure in 1-(Indan-1-on-oxy)-3-halogen-propan-2-ole überführen, wobei als Verdünnungs- oder Lösungsmittel neben an sich üblichen Solventien vorzugsweise ein aliphatischer oder cyclischer Äther, wie Diäthyläther, Tetrahydrofuran oder Dioxan, oder ein niederer Alkohol, wie Methanol, Äthanol oder Propanol verwendet werden. Andererseits können 1-(Indan-1-on-oxy)-3-halogen-propan-2-ole, insbesondere das 1-(Indan-1-on-4-oxy)-3-chlorpropan-2-ol und das 1-(Indan-1-on-4-oxy)-3-brom-propan-2-ol mit einer Base, wie einem Alkalimetallhydroxid, -carbonat, -hydrogencarbonat, -alkoholat oder -hydrid, einem tertiär-organischen Amin, wie Pyridin, Piperidin oder einem tertiären aliphatischen Amin, wie Trimethylamin oder Triäthylamin, in 1-(Indan-1-on-4-oxy)-2,3-epoxypropan umgewandelt werden. Diese Reaktionen können bei Raumtemperaturen durchgeführt werden und oder Wärmezufuhr, z. B. durch Erwärmen auf 60 bis 120 °C, beschleunigt oder abgeschlossen werden.

Die Reaktion kann unter Atmosphärendruck oder in einem geschlossenen Gefäß unter erhöhtem Druck, gegebenenfalls bei gleichzeitigem Erwärmen, durchgeführt werden. Die Ausgangsstoffe für diese Umwandlung können zuvor isoliert oder im Reaktionsgemisch erzeugt und ohne weitere Isolierung und Reinigung unmittelbar weiterverarbeitet werden.

Die erfindungsgemäßen Verbindungen der Formel I, in denen $R^1$ eine Hydroxygruppe bedeutet, weisen ein Chiralitätszentrum auf und werden als Racemate erhalten, die durch bekannte Methoden, beispielsweise durch Bildung diastereomerer Salze mit optisch aktiven Hilfssäuren, wie Dibenzoylweinsäure, Campher-10-sulfonsaüre, Ditoluylweinsäure oder 3-Brom-campher-8-sulfonsäure, in die optisch aktiven Antipoden getrennt werden können.

Die erhaltenen erfindungsgemäßen Verbindungen werden gegebenenfalls nach bekannter Verfahrensweise in das Säureadditionssalz einer physiologisch verträglichen Säure überführt. Als übliche physiologisch verträgliche organische oder anorganische Säuren kommen beispielsweise in Betracht Salzsäure, Bromwasserstoffsäure, Phosphorsäure oder Schwefelsäure und als organische Säure beispielsweise Oxalsäure, Maleinsäure, Fumarsäure, Milchsäure, Weinsäure, Apfelsäure, Zitronensäure, Salicylsäure, Adipinsäure oder Benzoesäure oder können aus Fortschritte der Arzneimittelforschung Band *10*, Seite 224 bis 225, Birkhäuser-Verlag, Basel und Stuttgart, 1966 oder Journal of Pharmaceutical Science, Volume 66, Seiten 1 bis 5 (1977) entnommen werden.

Der erfindungsgemäßen Verbindungen und ihre physiologisch verträglichen Säureadditionssalze weisen wertvolle pharmakologische Eigenschaften auf. Sie können als Sedativa, Tranquilizer, Neuroleptica oder als Mittel bei der Behandlung der Hypertonie verwendet werden.

Sie eignen sich mit bevorzugter sedativ/transquillisierender und anti-monaminerger Wirkung als Sedativa/Hypnotika oder als Tranquilizer bzw. Neuroleptica. Andere erfindungsgemäße Verbindungen eignen sich wegen ihrer relativ selektiven blutdrucksenkenden Wirksamkeit zur Behandlung der Hypertonie und zur Entlastung des Herzen bei Herzinsuffizienz.

Zu Nachweis der pharmakologischen Wirkungen wurden folgende Methoden verwendet :

1. Sedative Wirkung

4-8 Gruppen von jeweils 3 weiblichen NMRI-Mäusen erhalten die Substanz oral appliziert. Die photoelektrische Bestimmung der durch eine neue Umgebung induzierten Orientierungshypermotilität erfolgt 30 min nach der Applikation der Substanzen für eine Dauer von 30 min.

Als ED 50 wird die Dosis bestimmt, welche im Vergleich zu Placebo-behandelten Kontrolltieren eine Abnahme der Orientierungshypermotilität um 50 % bewirkt.

2. Hypotensive Wirkung

Die blutdrucksenkende Wirkung wurde an narkotisierten Ratten (Stamm : Sprague Dawley, Gewicht : 230-280 g) in Urethan-Narkose (1,78 g/kg i. p.) nachgewiesen. Der Blutdruck wurde in der Arteria carotis gemessen. Die Substanzapplikation erfolgte in eine Vena jugularis (wäßrige Lösung, 1 ml/kg). Als ED 20 % wurden aus der linearen Regression zwischen log Dosis (mg/kg) und relativer Blutdrucksenkung (Δ%) die Dosen ermittelt, welche eine 20 %ige Blutdrucksenkung bewirken.

Aus Tabelle 1 geht hervor, daß die Substanzen der Beispiele 12, 16, 1, 4 und 23 sedativ wirken. Die wirksamen Dosen sind etwas größer als die von Chlorpromazin, so daß die Wirkungsstärke der erfindungsgemäßen Substanzen zwischen 22 % (Beispiel 14) und 69 % (Beispiel 1) der Referenzsubstanz beträgt. Dagegen ist die für Sedativa/Neuroleptika unerwünschte Nebenwirkung « Blutdrucksenkung » deutlich geringer ausgeprägt als bei Chlorpromazin. Die hypotensiv wirksamen Dosen sind 6- (Beispiel 23) bis 300-mal (Beispiel 16) größer als die der Referenzsubstanz.

Bei den Verbindungen der Tabelle 1 ist danach ein günstigeres Verhältnis zwischen der erwünschten zentral dämpfenden (Motilitätshemmung) und der unerwünschten (Blutdrucksenkung) Wirkung nachgewiesen.

Andererseits wirken einige der beanspruchten Verbindungen stark hypotensiv (Tabelle 2). Nach den blutdrucksenkenden Dosen (ED 20 %) beträgt die Wirksamkeit 16- (Beispiel 9) bis 89 % (Beispiel 11) von Chlorpromazin bzw. ist Beispiel 17, 2,5-mal stärker wirksam als Chlorpromazin. Die sedative Wirkung dieser Substanzen ist im Vergleich zur Referenzsubstanz wesentlich schwächer. Eine Motilitätshemmung um 50 % bewirken 5- (Beispiel 11) bis 20-mal (Beispiel 9 und 17) größere Dosen. Die Quotienten aus den effektiven Dosen für die Motilitätshemmung (ED 50 %) und Blutdrucksenkung (ED 20 %) betragen zwischen 183 (Beispiel 14) und 4 077 (Beispiel 17) und übertreffen damit die Vergleichssubstanz Chlorpromazin (Q = 81) sehr deutlich.

Die vorliegenden Ergebnisse zeigen, daß sich innerhalb der erfindungsgemäßen Verbindungen 2 Gruppen mit unterschiedlichem Wirkprofil auszeichnen. Eine Gruppe von Substanzen mit bevorzugter zentralnervöser und geringer hypotensiver Wirkung ist für die Verwendung als Neuroleptika und in niederer Dosierung als Sedativa/Hypnotika bzw. Tranquilizer geeignet. Die 2. Gruppe mit ausgeprägter hypotensiver Wirkung ist wegen der gegenüber Chlorpromazin deutlich verminderten zentral dämpfenden Wirkung zur Behandlung der Hypertronie geeignet.

Tabelle 1
Sedative und hypotensive Wirkung

| Substanz | Motilitätshemmung[a] ED 50 % | | Blutdrucksenkung[b] ED 20 % | |
|---|---|---|---|---|
| Beispiel Nr. | mg/kg | R.W.[c] | mg/kg | R.W. |
| 12 | 9,44 | 0,22 | 1,00 | 0,026 |
| 16 | 5,76 | 0,37 | 7,91 | 0,003 |
| 1 | 3,05 | 0,69 | 1,07 | 0,024 |
| 4 | 3,56 | 0,59 | 0,189 | 0,14 |
| 23 | 5,03 | 0,42 | 0,161 | 0,16 |
| Chlorpromazin | 2,11 | 1,00 | 0,0260 | 1,00 |

[a]) Maus, Applikation : per os
[b]) Ratte, Urethan-Narkose, Applikation : i. v.
[c]) Relative Wirksamkeit ; Chlorpromazin = 1,00.

Tabelle 2
Hypotensive und sedative Wirkung

| Substanz | Blutdrucksenkung[a] ED 20 % | | Motilitätshemmung[b] ED 50 % | | Q[c] |
|---|---|---|---|---|---|
| Beispiel Nr. | mg/kg | R.W.[d] | mg/kg | R.W. | |
| 11 | 0,0293 | 0,89 | 11,03 | 0,19 | 376 |
| 14 | 0,0722 | 0,36 | 13,18 | 0,16 | 183 |
| 20 | 0,0570 | 0,46 | 18,98 | 0,11 | 333 |
| 17 | 0,0104 | 2,50 | 42,4 | 0,05 | 4077 |
| 9 | 0,158 | 0,16 | 46,4 | 0,05 | 294 |
| Chlorpromazin | 0,0260 | 1,00 | 2,11 | 1,00 | 81 |

[a]) Ratte, Urethan-Narkose, Applikation : i. v.
[b]) Maus, Applikation : per os.
[c]) ED 50 % Motilitätshemmung/ED 20 % Blutdrucksenkung.
[d]) Relative Wirksamkeit : Chlorpromazin = 1,00.

Gegenstand der vorliegenden Erfindung sind demnach auch therapeutische Mittel oder Zubereitungen, die neben den üblichen Träger- und Verdünnungsmitteln eine Verbindung der Formel (I) oder ihre physiologisch verträglichen Säureadditionssalze als Wirkstoff enthalten, sowie die Verwendung der neuen Verbindungen zu therapeutischen Zwecken.

Die therapeutischen Mittel bzw. Zubereitungen werden mit den üblichen festen oder flüssigen Trägerstoffen oder Verdünnungsmitteln und den üblicherweise verwendeten pharmazeutisch-technischen Hilfsstoffen entsprechend der gewünschten Applikationsart mit einer geeigneten Dosierung in

bekannter Weise hergestellt. Dabei kommen beim Menschen bei oraler Gabe Dosen von 10 bis 200 mg und bei intravenöser Gabe 10 bis 100 mg in Betracht.

Die bevorzugten Zubereitungen bestehen in einer Darreichungsform, die zur oralen Applikation geeignet ist. Solche Darreichungsformen sind beispielsweise Tabletten, Filmtabletten, Dragees, Kapseln, Pillen, Pulver, Lösungen oder Suspensionen oder Depotformen.

Zur praktischen Anwendung werden die erfindungsgemäß zu verwendenden Verbindungen mit den in der galenischen Pharmazie üblichen flüssigen oder festen Trägerstoffen verarbeitet. Die entsprechenden Tabletten können beispielsweise durch Mischen des Wirkstoffs mit bekannten Hilfsstoffen, beispielsweise inerten Verdünnungsmitteln, wie Dextrose, Zucker, Sorbit, Polyvinylpyrrolidon, Mannit, Calciumcarbonat, Calciumphosphat oder Milchzucker, Sprengmitteln, wie Mais, Stärke, Alginsäure oder Polyvinylpyrrolidon, Bindemitteln wie Stärke oder Gelatine, Gleitmitteln, wie Magnesiumstearat oder Talkum, und/oder Mitteln zur Erzielung des Depoteffektes, wie Carboxypolymethylen, Carboxymethylcellulose, Celluloseacetatphthalat oder Polyvinylacetat, erhalten werden. Die Tabletten können auch aus mehreren Schichten bestehen.

Entsprechend können Dragees durch Überziehen von analog den Tabletten hergestellten Kernen mit üblicherweise in Drageeüberzügen verwendeten Mitteln, beispielsweise Polyvinylpyrrolidon oder Schellack, Gummiarabicum, Talk, Titandioxid oder Zucker, hergestellt werden. Dabei kann auch die Drageehülle aus mehreren Schichten bestehen, wobei die oben bei den Tabletten erwähnten Hilfsstoffe verwendet werden können.

Die vorliegende Erfindung wird durch die nachfolgenden Ausführungsbeispiele näher erläutert.

I. Herstellung von Ausgangsverbindungen der Formel (II)

Beispiel I

1-(Indan-1-on-4-oxy)-2,3-epoxypropan

50 g 4-Hydroxy-indan-1-on (0,34 Mol) werden in 500 ml Isopropanol suspendiert und anschließend mit 13,5 (0,34 Mol) NaOH und 150 ml $H_2O$ versetzt. Dann werden 220 ml (2,8 Mol) Epichlorhydrin innerhalb einer halben Stunde zugegeben und anschließend 8 Stunden bei Raumtemperatur gut gerührt. Dann wird das Lösungsmittel und überschüssiges Epichlorhydrin unter vermindertem Druck abdestilliert, der Rückstand mehrmals mit Toluol extrahiert, die vereinigten Toluolphasen getrocknet und unter vermindertem Druck eingeengt. Der verbleibende Rückstand wird durch fraktionierte Destillation gereinigt. Man erhält 54 g (= 77,8 % Ausbeute) 1-(Indan-1-on-4-oxy)-2,3-epoxypropan vom $Kp_{0.1\text{-}0.2}$ 168-170 °C (kristallisiert beim Erkalten).

$C_{12}H_{12}O_3$ (204,23)
Ber.: 70,6 C 5,9 H 23,5 O
Gef.: 70,6 C 6,1 H 23,6 O

Beispiel II

1-(Indan-1-on-5-oxy)-2,3-epoxypropan

16 g 5-Hydroxy-indan-1-on (0,11 Mol) werden in 150 ml wasserfreiem Aceton gelöst, 30 g $K_2CO_3$ zugesetzt, 30 g Epibromhydrin (0,22 Mol) zugegeben und mit 100 mg NaJ versetzt. Der Ansatz wird unter Rühren 24 h lang unter Rückfluß gehalten. Anschließend wird filtriert und das Lösungsmittel unter vermindertem Druck abdestilliert. Es verbleiben 20 g 1-(Indan-1-on-5-oxy)-2,3-epoxypropan als Rohprodukt, welches ohne weitere Reinigung für die weitere Umsetzung verwendet wird.

Beispiel III

1-(Indan-1-on-7-oxy)-2,3-epoxypropan

30 g 7-Hydroxy-indan-1-on (0,2. Mol) werden in 250 ml Tetrahydrofuran gelöst, 28 g $K_2CO_3$ und 100 mg NaJ zugesetzt und anschließend 55 g Epibromhydrin unter Rühren bei Raumtemperatur zugetropft. Dann wird die Lösung unter gutem Rühren 30 h lang unter Rückfluß gehalten. Nach dem Erkalten wird filtriert, das Lösungsmittel und überschüssiges Epibromhydrin abdestilliert und der Rückstand zwischen Wasser und Toluol getrennt. Die Toluolphase wird mit schwach alkalischem Wasser gewaschen, dann getrocknet und am Ende eingeengt. Der verbleibende Rückstand kristallisiert beim stehen lassen nach einiger Zeit durch. Es werden auf diese Weise 26,5 g (= 69 % Ausbeute) 1-(Indan-1-on-7-oxy)-2,3-epoxypropan vom Fp 58-60 °C erhalten.

$C_{12}H_{12}O_3$ (204,23)
Ber.: 70,6 C 5,9 H 23,5 O
Gef.: 71,3 C 5,7 H —

Beispiel IV

1-(Indan-1-on-4-oxy)-3-chlor-propan

20 g 4-Hydroxy-indan-1-on (0,135 Mol) werden in 200 ml wasserfreiem DMF mit 30 g 1-Brom-3-chlorpropan (0,19 Mol) versetzt und in Gegenwart von 20 g wasserfreiem $K_2CO_3$ 12 h lang unter gutem Rühren bei 60 °C gehalten, danach noch 8 h bei Raumtemperatur nachgerührt. Anschließend wird das Lösungsmittel unter vermindertem Druck abdestilliert, der Rückstand zwischen Ether und schwach alkalischem Wasser getrennt. Die wäßrige Phase wird mehrmals mit Ether extrahiert, die Etherphasen vereinigt, mit $H_2O$ gewaschen, getrocknet und unter vermindertem Druck der Ether abdestilliert. Man erhält 24,3 g Rohprodukt als braunes Öl. Durch Behandlung mit Petrolether/Aceton wird der größte Teil des mitgebildeten 1.3-Bis-(indan-1-on-4-oxy)-propans ausgefällt, der verbleibende ölige Rückstand, der nach Entfernen des Petrolether/Aceton-Gemisches verbleibt, wird im Kugelrohr destilliert. Bei 220 °C gehen bei 0,2 mmHg 12,7 g 1-(Indan-1-on-4-oxy)-3-chlorpropan über.

$C_{12}H_{13}ClO_2$ (224,6)
Ber.: 64,2 C 5,8 H 15,8 Cl
Gef.: 64,4 C 5,9 H 15,3 Cl

Beispiel V

1-(Indan-1-on-7-oxy)-3-chlorpropan

5 g 7-Hydroxy-indan-1-on werden in 100 ml wasserfreiem Aceton gelöst und mit 8 g 1-Brom-3-chlorpropan und 7 g $K_2CO_3$ versetzt. Dann wird unter Rühren 3 Tage unter Rückfluß gehalten, anschließend filtriert, der Rückstand gut mit Aceton nachgewaschen und das Filtrat eingeengt. Der Rückstand wird zwischen Methylenchlorid und schwach alkalischem Wasser verteilt, die Wasserphase mehrere Male mit Methylenchlorid extrahiert, die vereinigten Methylenchloridphasen getrocknet und unter vermindertem Druck das Lösungsmittel abdestilliert. Es werden 6,5 g 1-(Indan-1-on-7-oxy)-3-chlorpropan als hellgelbes Öl isoliert, welches nach kurzer Zeit durchkristallisiert (Fp 53,5 °C).

$C_{12}H_{13}ClO_2$ (224,6)
Ber.: 64,2 C 5,8 H 15,8 Cl
Gef.: 64,6 C 6,2 H 15,5 Cl

II. Herstellung von erfindungsgemäßen Verbindungen

Beispiel 1

4-(3-[4-(4-Fluorphenyl)-1-piperazinyl]-2-hydroxy-propoxy)-indanon-1

7 g 1-(Indan-1-on-4-oxy)-2,3-epoxypropran und 6,1 g 4-Fluorphenylpiperazin werden in 100 ml n-Propanol gelöst und 8 h auf dem Wasserbad gehalten. Anschließend wird das Lösungsmittel unter vermindertem Druck abdestilliert. Der Rückstand wird in Methanol/Ether aufgenommen und mit ätherischer Salzsäure das Hydrochlorid gefällt. Das Kristallisat, welches sich u. U. erst beim Anreiben oder längerem Stehenlassen bildet, wird abgesaugt und aus Wasser unter Zusatz von Tierkohle umkristallisiert. Es werden so 5,1 g 4-(3-[4-(4-Fluorphenyl)-1-piperazinyl]-2-hydroxy-propxy)-indanon-1-monohydrochlorid vom Fp 206-208 °C isoliert.

$C_{22}H_{26}N_2ClFO_3$ (420,5)
Ber.: 62,7 C 6,1 H 6,6 N 4,5 F 8,4 Cl
Gef.: 62,5 C 6,1 H 6,8 N 4,3 F 8,4 Cl

Beispiel 2

5-(3-[4-(2-Chlorphenyl)-1-piperazinyl]-2-hydroxy-propoxy)-indanon-1-monohydrochlorid

6 g 1-(Indan-1-on-5-oxy)-2,3-epoxypropan und 6,7 g 2-Chlorphenylpiperazin werden in 100 ml Äthanol 10 h auf Rückflußtemperatur erhitzt. Das Lösungsmittel wird abdestilliert und der verbleibende Rückstand in wenig Methanol gelöst. Durch tropfenweise Zugabe von ätherischer Salzsäure wird das Hydrochlorid gefällt, welches anschließend aus Methanol/Ether umkristallisiert wird. Es werden so 2,7 g 5-(3-[4-(2-Chlorphenyl)-1-piperazinyl]-2-hydroxy-propoxy)-indan-1-on-monohydrochlorid vom Fp 249-252 °C erhalten.

$C_{22}H_{26}N_2Cl_2O_2$ (437,1)
Ber.: 60,4 C 5,9 H 6,4 N 16,2 Cl
Gef.: 60,5 C 6,1 H 6,6 N 16,1 Cl

## Beispiel 3

7-(3-[4-(4-Chlorphenyl)-1-piperazinyl]-2-hydroxy-propoxy)-indan-1-on

7 g 1-(Indan-1-on-7-oxy)-2,3-epoxypropan und 6,7 g 4-Chlorphenylpiperazin werden in 100 ml n-Propanol 3 Tage bei Raumtemperatur stehen gelassen. Anschließend wird das Lösungsmittel abdestilliert, der Rückstand in wenig Methanol gelöst und durch tropfenweise Zugabe von etherischer Salzsäure das Hydrochlorid gefällt, welches dann aus Äthanol umkristallisiert wird. Man erhält so 5,2 g 7-(3-[4-(4-Chlorphenyl)-1-piperazinyl]-2-hydroxy-propoxy)-indan-1-on-monohydrochlorid vom Fp 126-128 °C.

$C_{22}H_{26}N_2Cl_2O_3$ (437)
Ber.: 60,4 C 5,9 H 6,4 N 16,2 Cl
Gef.: 60,1 C 6,2 H 6,4 N 15,9 Cl

## Beispiel 4

4-(3-[4-(4-Fluorphenyl)-1-piperazinyl]-propoxy)-indan-1-on

5 g 1-(Indan-1-on-4-oxy)-3-chlorpropan und 4 g 4-Fluorphenyl-piperazin werden in 120 ml wasserfreiem DMF in Gegenwart von 5 g $K_2CO_3$ 8 h bei 120 °C unter gutem Rühren gehalten. Dann wird filtriert, die Lösung eingedampft und der Rückstand zwischen schwach alkalischem Wasser und Ether verteilt, die wäßrige Phase mehrere Male mit Ether nachextrahiert, die Etherphasen vereinigt, mit Wasser gewaschen, getrocknet und das Lösungsmittel abdestilliert. Es verbleiben 6,5 g braunes Öl, welches in wenig Methanol/Äther aufgenommen wird und durch tropfenweise Zugabe von ätherischer Salzsäure in das Hydrochlorid überführt wird.
Das Kristallisat wird abfiltriert und aus Aceton/Methanol/Ether umkristallisiert. Man erhält so 3,5 g 4-(3-[4-(4-Fluorphenyl)-1-piperazinyl]-propoxy)-indan-1-on-bis-hydrochlorid vom Fp 223 °C.

$C_{22}H_{27}N_2Cl_2FO_2$ (441)
Ber.: 59,8 C 6,1 H 6,3 N 16,1 Cl 4,3 F
Gef.: 60,1 C 6,2 H 6,1 N 15,9 Cl 4,2 F

Die in der nachfolgenden Tabelle aufgeführten Verbindungen werden entsprechend Beispiel 1 aus den entsprechenden Glycidäthern und den Phenylpiperazinen oder entsprechend Beispiel 4 aus den Indan-1-on-oxy-chloralkanen und den entsprechenden Phenylpiperazinen erhalten.

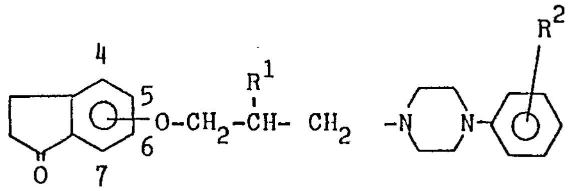

(Siehe Tabelle Seite 9 f.)

| Beispiel Nr. | Verknüpfung d. Seitenkette | $R^1$ | $R^2$ | Salzform | Fp [°C] |
|---|---|---|---|---|---|
| 5 | 4 | OH | H | 2 HCl | 209-210 |
| 6 | 4 | OH | 4-Cl | HCl | 225-226 |
| 7 | 4 | OH | 3-Cl | HCl | 189-191 |
| 8 | 4 | OH | 2-Cl | HCl | 205-206 |
| 9 | 4 | OH | 2-$CH_3$ | 2 HCl | 197 |
| 10 | 4 | OH | 4-$CH_3$ | HCl | 233-235 |
| 11 | 4 | OH | 2-$OCH_3$ | 2 HCl | 226-228 |
| 12 | 4 | OH | 3-$OCH_3$ | HCl | 180-183 |
| 13 | 4 | OH | 4-$OCH_3$ | HCl | 229-230 |
| 14 | 5 | OH | 2-$OCH_3$ | HCl | 227-228 |
| 15 | 5 | OH | 3-$OCH_3$ | HCl | 214-216 |
| 16 | 5 | OH | 4-$OCH_3$ | 2 HCl | 196-199 |

| Beispiel Nr. | Verknüpfung d. Seitenkette | $R^1$ | $R^2$ | n | Salzform | Fp [°C] |
|---|---|---|---|---|---|---|
| 17 | 7 | OH | 2-Cl | 1 | HCl | 187 |
| 18 | 7 | OH | 3-Cl | 1 | HCl | 170-171 |
| 19 | 7 | OH | 4-F | 1 | HCl | 112-114 |
| 20 | 7 | OH | 2-$OCH_3$ | 1 | 2 HCl | 188-189 |
| 21 | 7 | OH | 3-$OCH_3$ | 1 | HCl | 145-148 |
| 22 | 4 | H | 3-Cl | 1 | 2 HCl | 207 |
| 23 | 7 | H | 4-F | 1 | 2 HCl | 195 |
| 24 | 7 | H | 4-Cl | 1 | 2 HCl | 180-182 |

(Siehe Tabelle Seite 10 ff.)

| Beispiel | Analysen | | | | | | Bezeichnung |
|---|---|---|---|---|---|---|---|
| 5 | Ber.: | 60,1 C | 6,4 H | 6,4 N | 16,1 Cl | | 4-(3-[4-(Phenyl)-1-piperazinyl]-2--hydroxy-propoxy)-indanon-1 |
| | Gef.: | 59,8 C | 5,5 H | 6,5 N | 15,9 Cl | | |
| 6 | Ber.: | 60,4 C | 5,9 H | | 16,2 Cl | | 4-(3-[4-(4-Chlorphenyl)-1-piperazinyl]-2-hydroxy-propoxy)-indanon-1 |
| | Gef.: | 60,1 C | 5,9 H | | 16,0 Cl | | |
| 7 | Ber.: | 60,4 C | 5,9 H | 6,4 N | 16,2 Cl | | 4-(3-[4-(3-Chlorphenyl)-1-piperazinyl]-2-hydroxy-propoxy)-indanon-1 |
| | Gef.: | 59,9 C | 6,0 H | 6,2 N | 16,5 Cl | | |
| 8 | Ber.: | 60,4 C | 5,9 H | 6,4 N | 16,2 Cl | | 4-(3-[4-(2-Chlorphenyl)-1-piperazinyl]-2-hydroxy-propoxy)-indanon-1 |
| | Gef.: | 59,8 C | 5,9 H | 6,5 N | 16,2 Cl | | |
| 9 | Ber.: | 60,9 C | 6,6 H | 6,2 N | 15,6 Cl | | 4-(3-[4-(2-Methylphenyl)-1-piperazinyl]-2-hydroxy-propoxy)-indanon-1 |
| | Gef.: | 60,9 C | 7,0 H | 6,1 N | 15,1 Cl | | |
| 10 | Ber.: | 65,2 C | 7,2 H | 6,9 N | 8,8 Cl | | 4-(3-[4-(4-Methylphenyl)-1-piperazinyl]-2-hydroxy-propoxy)-indanon-1 |
| | Gef.: | 65,9 C | 6,9 H | 6,9 N | 8,5 Cl | | |
| 11 | Ber.: | 58,9 C | 6,2 H | 5,9 N | 15,1 Cl | | 4-(3-[4-(2-Methoxyphenyl)-1-piperazinyl]-2-hydroxy-propoxy)-indanon-1 |
| | Gef.: | 58,4 C | 6,4 H | 6,1 N | 15,3 Cl | | |
| 12 | Ber.: | 63,8 C | 6,7 H | 6,4 N | 8,2 Cl | | 4-(3-[4-(3-Methoxyphenyl)-1-piperazinyl]-2-hydroxy-propoxy)-indanon-1 |
| | Gef.: | 63,2 C | 7,0 H | 6,4 N | 8,2 Cl | | |
| 13 | Ber.: | 63,8 C | 6,7 H | 6,4 N | 8,2 Cl | | 4-(-[4-(4-Methoxyphenyl)-1-piperazinyl]-2-hydroxy-propoxy)-indanon-1 |
| | Gef.: | 63,1 C | 6,5 H | 6,1 N | 8,6 Cl | | |
| 14 | Ber.: | 63,8 C | 6,7 H | 6,4 N | 8,2 Cl | | 5-(3-[4-(2-Methoxyphenyl)-1-piperazinyl[-1-hydroxy-propoxy)-indanon-1 |
| | Gef.: | 63,5 C | 6,8 H | 6,6 N | 8,1 Cl | | |
| 15 | Ber.: | 63,8 C | 6,7 H | 6,4 N | 8,2 Cl | | 5-(3-[4-(3-Methoxyphenyl)-1-piperazinyl]-1-hydroxy-propoxy)-indanon-1 |
| | Gef.: | 62,9 C | 6,6 H | 6.1 N | 8,7 Cl | | |
| 16 | Ber.: | 58,9 C | 6,4 H | 5,9 N | 15,1 Cl | | 5-(3-[4-(4-Methoxyphenyl)-1-piperazinyl]-2-hydroxy-propoxy)-indanon-1 |
| | Gef.: | 58,7 C | 6,5 H | 5,6 N | 14,9 Cl | | |
| 17 | Ber.: | 60,4 C | 5,9 H | 6,4 N | 16,2 Cl | | 7-(3-[4-(2-Chlorphenyl)-1-piperazinyl]--2-hydroxy-propoxy)-indanon-1 |
| | Gef.: | 60,0 C | 6,0 H | 6,4 N | 15,9 Cl | | |
| 18 | Ber.: | 60,4 C | 5,9 H | 6,4 N | 16,2 Cl | | 7-(3-[4-(3-Chlorphenyl)-1-piperazinyl]--2-hydroxy-propoxy)-indanon-1 |
| | Gef.: | 59,9 C | 6,5 H | 6,1 N | 15,9 Cl | | |
| 19 | Ber.: | 62,7 C | 6,1 H | 6,6 N | 8,4 Cl | 4,5 F | 7-(3-[4-(4-Fluorphenyl)-1-piperazinyl]--2-hydroxy-propoxy)-indanon-1 |
| | Gef.: | 62,1 C | 6,7 H | 6,5 N | 8,3 Cl | 4,3 F | |
| 20 | Ber.: | 58,9 C | 6,4 H | 5,9 N | 15,1 Cl | | 7-(3-[4-(2-Methoxyphenyl)-1-piperazinyl]-2-hydroxy-propoxy)-indanon-1 |
| | Gef.: | 58,7 C | 6,6 H | 6,1 N | 14,9 Cl | | |

| Beispiel | Analysen | | | | | | Bezeichnung |
|---|---|---|---|---|---|---|---|
| 21 | Ber.: | 63,8 C | 6,7 H | 6,4 N | 8,2 Cl | | 7-(3-[4-(3-Methoxyphenyl)-1-piperazinyl]-2-hydroxy-propoxy)-indanon-1 |
| | Gef.: | 63,7 C | 7,0 H | 6,1 N | 7,8 Cl | | |
| 22 | Ber.: | 57,7 C | 5,9 H | 6,1 N | 23,3 Cl | | 4-(3-[4-(3-Chlorphenyl)-1-piperazinyl]-propoxy)-indanon-1 |
| | Gef.: | 57,7 C | 6,1 H | 6,1 N | 22,8 Cl | | |
| 23 | Ber.: | 59,8 C | 6,1 H | 6,3 N | 16,0 Cl | 4,3 F | 7-(3-[4-(4-Fluorphenyl)-1-piperazinyl]-propoxy)-indanon-1 |
| | Gef.: | 59,5 C | 6,1 H | 6,1 N | 15,8 Cl | 4,0 F | |
| 24 | Ber.: | 57,7 C | 5,9 H | 6,1 N | 23,2 Cl | | 7-(3-[4-(4-Chlorphenyl)-1-piperazinyl]-propoxy)-indanon-1 |
| | Gef.: | 58,1 C | 6,0 H | 6,0 N | 22,8 Cl | | |

Formulierungsbeispiele, die in üblicher Weise hergestellt werden :

1. Tabletten :

a)

| | |
|---|---|
| Ein Wirkstoff der Formel I | 40 mg |
| Lactose | 200 mg |
| Methylcellulose | 15 mg |
| Maisstärke | 50 mg |
| Talkum | 11 mg |
| Magnesiumstearat | 4 mg |
| | 320 mg |

b)

| | |
|---|---|
| Ein Wirkstoff der Formel I | 80 mg |
| Lactose | 178 mg |
| Avicel | 80 mg |
| Polywachs 6 000 | 20 mg |
| Magnesiumstearat | 2 mg |
| | 360 mg |

c)

| | |
|---|---|
| Ein Wirkstoff der Formel I | 50 mg |
| Polyvinylpyrrolidon (mittl. M.G. 25 000) | 170 mg |
| Polyäthylenglykol (mittl. M.G. 4 000) | 14 mg |
| Hydroxypropylmethylcellulose | 40 mg |
| Talkum | 4 mg |
| Magnesiumstearat | 2 mg |
| | 280 mg |

Der Wirkstoff wird mit Polyvinylpyrrolidon in 10 %iger wäßriger Lösung befeuchtet, durch ein Sieb mit der lichten Maschenweite 1,0 mm getrieben und bei 50 °C getrocknet. Dieses Granulat wird mit Polyäthylenglykol (mittl. M.G. 4 000), Hydroxypropylmethylcellulose, Talkum und Magnesiumstearat vermischt und zu Tabletten a 280 mg verpreßt.

2. Beispiel für Dragees :

| | |
|---|---|
| Ein Wirkstoff der Formel I | 60 mg |
| Lactose | 90 mg |
| Maisstärke | 60 mg |
| Polyvinylpyrrolidon | 6 mg |
| Magnesiumstearat | 1 mg |
| | 217 mg |

Die Mischung der Wirkstoffsubstanz mit Lactose und Maisstärke wird mit einer 8 %igen wäßrigen Lösung des Polyvinylpyrrolidons durch Sieb 1,5 mm granuliert, bei 50 °C getrocknet und nochmals durch Sieb 1,0 mm gerieben. Das so erhaltene Granulat wird mit Magnesiumstearat und zu Dragеekernen verpreßt. Die erhaltenen Drageekerne werden in üblicher Weise mit einer Hülle überzogen, die im wesentlichen aus Zucker und Talkum besteht.

3. Kapselformulierung :

| | |
|---|---|
| Ein Wirkstoff der Formel I | 5,0 mg |
| Magnesiumstearat | 2,0 mg |
| Milchzucker | 19,3 mg |

4. Injektionslösung :

| | |
|---|---|
| Ein Wirkstoff der Formel I | 10 mg |
| Natriumchlorid destilliertes Wasser, q. s. auf 1,0 ml | 9 mg |

**Ansprüche** (für die Vertragsstaaten : BE, CH, DE, FR, GB, IT, LI, LU, NL, SE)

1. Verbindungen der allgemeinen Formel (I)

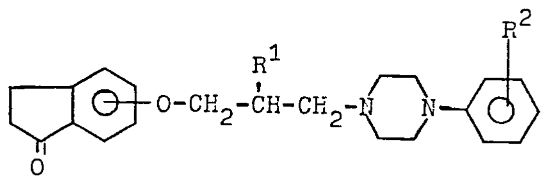

(I)

in der $R^1$ ein Wasserstoffatom oder eine Hydroxylgruppe und $R^2$ ein Chlor- oder Fluoratom, eine Methoxy, oder Methylgruppe bedeuten, und die Oxyalkylpiperazinseitenkette in der 4-, 5- oder 7-Stellung des Indanon-1-ringsystems sich befindet, und ihre physiologisch verträglichen Säureadditionssalze.

2. 4-(3-[4-(4-Fluorphenyl)-1-piperazinyl]-2-hydroxy-propoxy)-indanon-1 nach Anspruch 1.

3. 7-(3-[4-(2-Chlorphenyl)-1-piperazinyl]-2-hydroxy-propoxy)-indanon-1 nach Anspruch 1.

4. Verfahren zur Herstellung von Verbindungen der allgemeinen Formel I nach Anspruch 1, dadurch gekennzeichnet, daß man ein Indanon-Derivat der Formel (II)

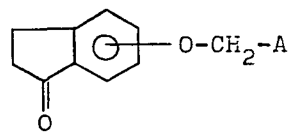

(II)

in der A den Rest

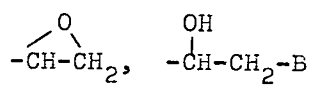 oder $-CH_2-B$

bedeutet und wobei B für eine nukleofuge Abgangsgruppe, mit einem Piperazinderivat der allgemeinen Formel (III)

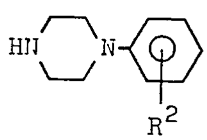

(III)

in der $R^2$ die für die Formel (I) angegebenen Bedeutungen hat, zweckmäßigerweise in einem Lösungsmittel und ggf. in Gegenwart eines säurebindenden Mittels in an sich bekannter Weise umsetzt und die erhaltene Verbindung ggf. in das Säureadditionssalz einer physiologisch verträglichen Säure überführt.

5. Therapeutische Mittel, gekennzeichnet durch einen Gehalt an einer Verbindung der Formel I oder ihres physiologisch verträglichen Säureadditionssalzes als Wirkstoff neben üblichen Trägerstoffen und Verdünnungsmitteln.

6. Verbindungen der allgemeinen Formel (I) gemäß Anspruch 1 zur Verwendung bei der Bekämpfung von Krankheiten.

**Ansprüche** (für den Vertragsstaat AT)

1. Verfahren zur Herstellung von Verbindungen der allgemeinen Formel (I)

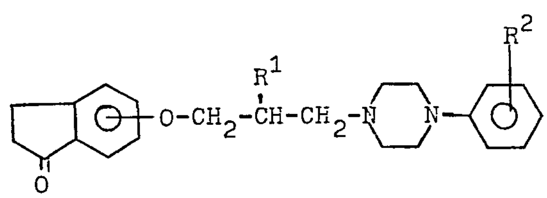

(I)

in der $R^1$ ein Wasserstoffatom oder eine Hydroxylgruppe und $R^2$ ein Chlor- oder Fluoratom, eine Methoxy- oder Methylgruppe bedeuten, und die Oxyalkylpiperazinseitenkette in der 4-, 5- oder 7-Stellung des Indanon-1-ringsystems sich befindet, und deren physiologisch verträglichen Säureadditionssalzen, dadurch gekennzeichnet, daß man ein Indanon-Derivat der Formel (II)

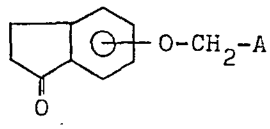

(II)

in der A den Rest

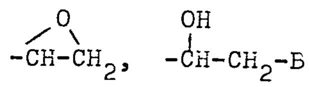 oder $-CH_2-B$

bedeutet und wobei B für eine nukleofuge Abgangsgruppe mit einem Piperazinderivat der allgemeinen Formel (III)

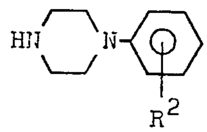

(III)

in der $R^2$ die für die Formel (I) angegebenen Bedeutungen hat, zweckmäßigerweise in einem Lösungsmittel und ggf. in Gegenwart eines säurebindenden Mittels in an sich bekannter Weise umsetzt und die erhaltene Verbindung ggf. in das Säureadditionssalz einer physiologisch verträglichen Säure überführt.

2. Verfahren nach Anspruch 1, dadurch gekennzeichnet, daß man 4-(3-[4-(4-Fluorphenyl)-1-piperazinyl]-2-hydroxy-propoxy)-indanon-1 herstellt.

3. Verfahren nach Anspruch 1, dadurch gekennzeichnet, daß man 7-(3-[4-(2-chlorphenyl)-1-piperazinyl]-2-hydroxy-propoxy)-indanon-1 herstellt.

**Claims** (for the Contracting States : BE, CH, DE, FR, GB, IT, LI, LU, NL, SE)

1. A compound of the general formula (I)

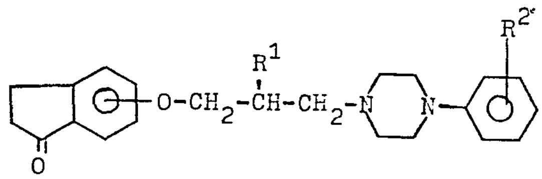

(I)

where $R^1$ is hydrogen or hydroxyl, $R^2$ is chlorine, fluorine, methoxy or methyl and the oxyalkyl-piperazine side chain is in the 4-, 5- or 7-position of the indan-1-one ring system, and its physiologically tolerated addition salts with acids.

2. 4-(3-[4-(4-Fluorophenyl)-piperazin-1-yl]-2-hydroxy-propoxy)-indan-1-one, as claimed in claim 1.

3. 7-(3-[4-(2-Chlorophenyl)-piperazin-1-yl]-2-hydroxy-propoxy)-indan-1-one, as claimed in claim 1.

4. A process for the preparation of a compound of the general formula I as claimed in claim 1, wherein an indanone derivative of the formula (II)

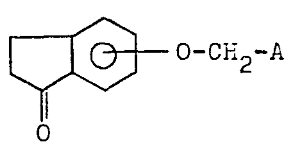

(II)

where A is

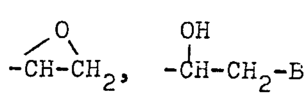 or $-CH_2-B$

B being a nucleofugic leaving group, is reacted, in a conventional manner, with a piperazine derivative of the general formula (III)

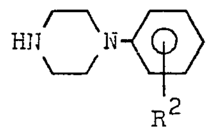

(III)

where $R^2$ has the meanings given for formula (I), advantageously in a solvent and in the presence or absence of an acid acceptor, and, if desired, the resulting compound is converted into its addition salt with a physiologically tolerated acid.

5. A therapeutic agent, which contains a compound of the formula I, or one of its physiologically tolerated addition salts with an acid, as the active compound, in addition to conventional carriers and diluents.

6. A compound of the general formula (I) as claimed in claim 1 for use in the treatment of disorders.

**Claims** (for the Contracting State AT)

1. A process for the preparation of a compound of the general formula (I)

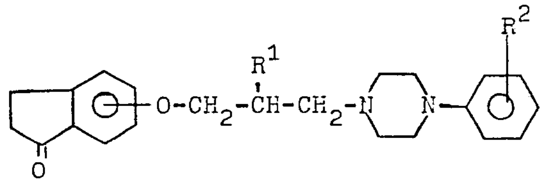

(I)

where $R^1$ is hydrogen or hydroxyl, $R^2$ is chlorine, fluorine, methoxy or methyl and the oxyalkyl-piperazine side chain is in the 4-, 5- or 7-position of the indan-1-one ring system, and its physiologically tolerated addition salts with acids, wherein an indanone derivative of the formula (II)

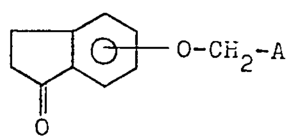

(II)

where A is

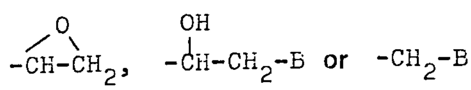

B being a nucleofugic leaving group, is reacted, in a conventional manner, with a piperazine derivative of the general formula (III)

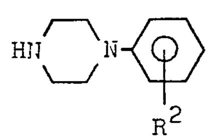

(III)

where $R^2$ has the meanings given for formula (I), advantageously in a solvent and in the presence or absence of an acid acceptor, and, if desired, the resulting compound is converted into its addition salt with a physiologically tolerated acid.

2. A process as claimed in claim 1, wherein 4-(3-[4-(4-fluorophenyl)-piperazin-1-yl]-2-hydroxy-propoxy)-indan-1-one is prepared.

3. A process as claimed in claim 1, wherein 7-(3-[4-(2-chlorophenyl)-piperazin-1-yl]-2-hydroxy-propoxy)-indan-1-one is prepared.

**Revendications** (pour les Etats contractants : BE, CH, DE, FR, GB, IT, LI, LU, NL, SE)

1. Composés de formule générale (I)

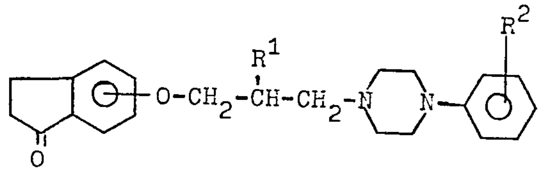

(I)

dans laquelle $R^1$ représente un atome d'hydrogène ou un groupe hydroxyle et $R^2$ un atome de chlore ou de fluor, un groupe méthoxy ou méthyle, et la chaîne latérale oxyalkylpipérazine se trouve en position 4-, 5- ou 7- du système cyclique indanone-1, et leurs sels d'addition d'acide tolérables physiologiquement.

2. 4-(3-[4-(4-fluorophényl)-1-pipérazinyl]-2-hydroxy-propoxy-indanone-1 selon la revendication 1.

3. 7-(3-[4-(2-chlorophényl)-1-pipérazinyl]-2-hydroxy-propoxy)-indanone-1 selon la revendication 1.

4. Procédé de préparation de composés de formule générale I selon la revendication 1, caractérisé par le fait que l'on fait réagir, de manière connue en soi, avantageusement dans un solvant et éventuellement en présence d'un agent liant d'acide, un dérivé d'indanone de formule (II)

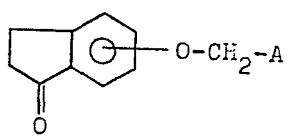

(II)

dans laquelle A signifie le reste

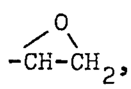 , 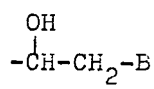 ou $-CH_2-B$

et B représentant un groupe éliminable nucléofuge, avec un dérivé de pipérazine de formule générale (III)

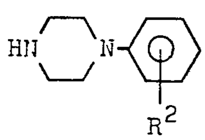

(III)

dans laquelle $R^2$ a la signification donnée pour la formule (I), et l'on transforme le composé obtenu éventuellement dans le sel d'addition d'un acide tolérable physiologiquement.

5. Agent thérapeutique, caractérisé par une teneur, comme principe actif, en un composé de formule I ou son sel d'addition d'acide tolérable physiologiquement, outre des véhicules et diluants usuels.

6. Composés de formule générale (I) selon la revendication 1 pour l'utilisation dans la lutte contre les maladies.

**Revendications** (pour l'Etat contractant AT)

1. Procédé de préparation de composés de formule générale (I)

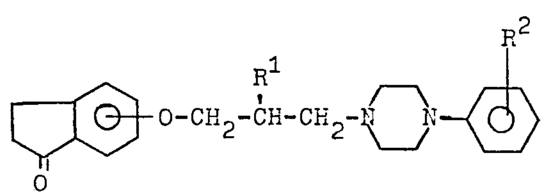

(I)

dans laquelle $R^1$ représente un atome d'hydrogène ou un groupe hydroxyle, et $R^2$ un atome de chlore ou de fluor, un groupe méthoxy ou méthyle, et la chaîne latérale oxyalkylpipérazine se trouve en position 4-, 5- ou 7- du système cyclique indanone-1, et leurs sels d'addition d'acide tolérables physiologiquement, caractérisé par le fait que l'on fait réagir, de manière connue en soi, avantageusement dans un solvant et éventuellement en présence d'un agent liant d'acide, un dérivé d'indanone de formule (II)

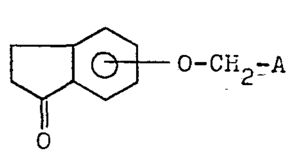

(II)

dans laquelle A signifie le reste

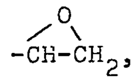, 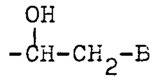 ou $-CH_2-B$

et B représentant un groupe éliminable nucléofuge, avec un dérivé de pipérazine de formule générale (III)

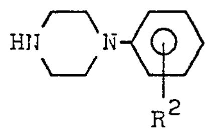

(III)

dans laquelle $R^2$ a la signification donnée pour la formule (I), et l'on transforme le composé obtenu éventuellement dans le sel d'addition d'un acide tolérable physiologiquement.

2. Procédé selon la revendication 1, caractérisé par le fait qu'on prépare du 4-(3-[4-(4-fluorophényl)-1-pipérazinyl]-2-hydroxy-propoxy)-indanone-1.

3. Procédé selon la revendication 1, caractérisé par le fait qu'on prépare du 7-(3-[4-(2-chlorophényl)-1-pipérazinyl]-2-hydroxy-propoxy)-indanone-1.